# EUROPÄISCHE PATENTSCHRIFT

(11) **EP 2 805 748 B1**
(45) Veröffentlichungstag und Bekanntmachung des Hinweises auf die Patenterteilung: **26.09.2018**
(21) Anmeldenummer: 13169183.4
(22) Anmeldetag: 24.05.2013
(51) Int. Cl.: A62B 9/02, A62B 18/10, A61M 16/06, A61M 16/20

(54) **Atemmaske mit Notatemventil**
Respiration mask with emergency respiration valve
Masque respiratoire doté d'une soupape de respiration de secours

(43) Veröffentlichungstag der Anmeldung: 26.11.2014
(73) Patentinhaber: Drägerwerk AG & Co. KGaA, 23558 Lübeck (DE)
(72) Erfinder: Wallnewitz, Oliver, 23556 Lübeck (DE); Kaszás, Helena, 23627 Groß Grönau (DE); Soltész, Krisztina, 23564 Lübeck (DE)
(74) Vertreter: Uexküll & Stolberg

(56) Entgegenhaltungen:
- WO-A2-01/78838
- GB-A- 1 553 437
- US-A1- 2006 249 158

## Beschreibung

Die vorliegende Erfindung betrifft eine Atemmaske mit einem Maskenkörper, der einen zu einer Seite offenen Hohlraum umgibt und der zur Anlage an ein menschliches Gesicht um dessen Mund und/oder Nase vorgesehen ist, wobei Mund und/oder Nase in dem Hohlraum angeordnet werden können, mit einem Zuführanschluss zum Anschluss einer Atemgaszuführeinrichtung für eine Zufuhr von Gas in den Hohlraum, mit einer Ventilanordnung, die ein Exspirationsventil zum Steuern des Durchlasses von Gas aus dem Hohlraum in die die Atemmaske umgebende Atmosphäre und ein Notatemventil zum Steuern des Durchlasses von Gas aus der die Atemmaske umgebenden Atmosphäre in den Hohlraum aufweist, wobei das Exspirationsventil einen Verschlusskörper zum Verschließen einer Exspirationsventilöffnung aufweist und das Exspirationsventil derart angepasst ist, dass es öffnet, wenn der Druck im Hohlraum um einen vorgegebenen Betrag über dem Druck der die Atemmaske umgebenden Atmosphäre liegt, wobei das Notatemventil derart angepasst ist, dass es öffnet, wenn der Druck im Hohlraum um einen vorgegebenen Betrag unter dem Druck der die Atemmaske umgebenden Atmosphäre liegt, wobei das Notatemventil ein Blendenelement aufweist, das mit einer Blendenöffnung versehen ist, die eine Gasverbindung zwischen dem Hohlraum und der die Atemmaske umgebenden Atmosphäre bildet, wobei das Notatemventil ein Verschlusselement mit einer Verschlussfläche aufweist, das derart flexibel ausgebildet ist, dass es zwischen einer Öffnungsstellung, in der die Verschlussfläche von dem Blendenelement beabstandet ist, sodass ein Strömen von Gas durch die Öffnung zugelassen wird, und einer Schließstellung verformbar ist, in der die Verschlussfläche an dem Blendenelement anliegt, sodass das Verschlusselement die Blendenöffnung zur die Atemmaske umgebenden Atmosphäre verschließt und ein Strömen von Gas durch die Blendenöffnung verhindert wird. Ferner betrifft die vorliegende Erfindung ein Beatmungssystem mit einer Atemmaske.

Derartige Atemmasken werden insbesondere dazu verwendet, einen Patienten mit Sauerstoff zu versorgen, wobei der Zuführanschluss mit einer Atemgas- und insbesondere einer Sauerstoffversorgung verbunden ist, die ein Einatemventil aufweist, das einen Strom von Atemgas in den Hohlraum steuert und das derart ausgestaltet ist, das es dann öffnet, wenn der Druck in dem Hohlraum um einen vorgegebenen Betrag unter dem Druck in der Atemgasversorgung liegt. Dabei ist für gewöhnlich ein Gas- oder Sauerstoffbeutel in der Atemgasversorgung vorgesehen, der wiederum an die Sauerstoffversorgung angeschlossen ist. Damit dient der Sauerstoffbeutel als Puffer, und es ist nicht erforderlich, dass die Sauerstoffversorgung einen so großen Volumenstrom bereitstellt, wie es in der Inspirationsphase, wenn der Patient einatmet, erforderlich wäre. Vielmehr ist es ausreichend, dass die Sauerstoffversorgung einen Volumenstrom bereitstellt, der dem zeitlichen Mittel der Sauerstoffaufnahme des Patienten entspricht.

Darüber hinaus ist ein Exspirationsventil in der Atemmaske vorgesehen, das öffnet, wenn sich im Inneren des Hohlraums in der Ausatemphase ein Druck aufbaut, der höher als der Umgebungsdruck ist. Das Exspirationsventil ist also derart aufgebaut, dass es lediglich eine Strömung von dem Hohlraum nach außen zulässt, in dem Hohlraum also ein im Vergleich zur Umgebung höherer Druck herrschen muss.

Die WO 01/78838 A2 beschreibt in diesem Zusammenhang eine Atemmaske, die eine Ventilanordnung mit einem Einatemventil und einem Exspirationsventil umfasst, wobei die Ventilanordnung lösbar an der Atemmaske befestigt ist. Die Atemmaske weist ferner einen Einlass auf, mit dem die Atemmaske mit einem Sauerstoffbeutel zum Einatmen verbunden wird, und einen Auslass zur anschließenden Exspiration. Zusätzlich sind an dem Gehäuse der Atemmaske Öffnungen vorgesehen.

Solche Atemmasken, die lediglich über ein Exspirationsventil verfügen, in Verbindung mit einem Einatemventil in der Atemgasversorgung sind jedoch mit dem Nachteil verbunden, dass die Gefahr besteht, dass der Patient nicht ausreichend mit Atemgas versorgt wird, wenn der Volumenstrom der Atemgasversorgung hinter dem Bedarf des Patienten zurückbleibt oder ganz ausfällt. Dann kann es passieren, dass das in dem Sauerstoffbeutel vorhandene Volumen von dem Patienten verbraucht wird und dieser dann nicht mehr mit weiterem Atemgas versorgt wird.

Um dieses Problem zu vermeiden, ist es aus der EP 1 854 494 B1 bekannt, neben dem Einatem- und dem Exspirationsventil noch ein Notatemventil vorzusehen. Das Notatemventil sieht eine Verbindung zwischen dem Hohlraum und der die Atemmaske umgebenden Umgebung vor und ist derart aufgebaut, dass es erst dann öffnet, wenn im Hohlraum der Atemmaske ein Druck herrscht, der niedriger ist als der Druck, bei dem bereits das Einatemventil öffnet. Dies führt dazu, dass das Notatemventil nur dann öffnet, wenn der Patient in der Einatemphase kein Atemgas aus der Atemgasversorgung mehr erhält und sich daher in dem Hohlraum ein niedriger Druck aufbaut als es nötig ist, um das Einatemventil zu öffnen.

Bei dem in der EP 1 854 494 B1, von der die vorliegende Erfindung ausgeht, beschriebenen Aufbau sind der Ventilkörper, der das Einatemventil schließt oder öffnet, der Ventilkörper, der das Exspirationsventil schließt oder öffnet, und der Ventilkörper des Notatemventils einstückig aus einem flächigen flexiblen Material ausgebildet. Dies ist jedoch mit dem Nachteil verbunden, dass die Druckschwellen, bei denen das Inspirationsventil und das Notatemventil öffnen und die aufeinander abgestimmt sein müssen, nur mit sehr ungenügender Präzision eingestellt werden können.

Daher ist es ausgehend von dem Stand der Technik die Aufgabe der vorliegenden Erfindung, eine Atemgasmaske bereitzustellen, bei der die Druckschwellen, bei denen das Exspirations- und das Notatemventil jeweils öffnen, mit hoher Genauigkeit aufeinander abgestimmt werden können und die einfach montiert werden kann.

Erfindungsgemäß wird diese Aufgabe dadurch gelöst, dass das Verschlusselement des Notatemventils als separates Bauteil der Ventilanordnung ausgebildet ist, dass das Blendenelement als separates Bauteil ausgebildet ist, das auf der von dem Hohlraum wegweisenden Seite an dem Maskenkörper befestigt ist und dass der Verschlusskörper des Exspirationsventils auf der von dem Hohlraum wegweisenden Seite zwischen dem Blendenelement und dem Maskenkörper gehaltert ist.

Dadurch, dass das Verschlusselement des Notatemventils als separates Bauteil ausgebildet ist, kann dieses im Hinblick auf dessen Material und die damit verbundenen elastischen Eigenschaften und dessen Abmessungen vollkommen unabhängig von dem Verschlusselement des Exspirationsventils ausgestaltet werden. Somit ergibt sich eine große Freiheit und damit auch die Möglichkeit, die Schwelle, bei der das Notatemventil öffnet, genau einzustellen.

Vorzugsweise weist das Verschlusselement ein erstes Verbindungselement auf, wobei das Blendenelement mit einem zweiten Verbindungselement zum Eingriff mit dem ersten Verbindungselement versehen ist. Insbesondere kann das erste Verbindungselement von der Verschlussfläche umgeben sein, wobei das zweite Verbindungselement innerhalb der Blendenöffnung angeordnet ist und einen sich radial zum Rand der Blendenöffnung erstreckenden Arm aufweist. Eines der Verbindungselemente kann dabei als sich von der Blendenöffnung weg erstreckender Stift ausgebildet sein, während das andere der Verbindungselemente als Aufnahmeöffnung für einen Eingriff mit dem Stift ausgebildet ist. Ein solcher Aufbau lässt eine einfache Montage des Verschlusselements zu. Außerdem ist das Verschlusselement leicht austauschbar. Hierbei ist es einerseits möglich, dass der Stift an dem Verschlusselement und die Aufnahmeöffnung in einer Aufnahmebuchse ausgebildet sind, die an wenigstens einem sich vom Rand der Blendenöffnung ersteckenden Arm vorgesehen ist. Umgekehrt kann der Stift aber auch an dem wenigstens einen Arm vorgesehen sein, während die Aufnahmeöffnung in dem Verschlusselement ausgebildet ist.

Erfindungsgemäß ist das Blendenelement als separates Bauteil ausgebildet, das auf der von dem Hohlraum wegweisenden Seite an dem Maskenkörper befestigt ist, wobei der Verschlusskörper des Exspirationsventils auf der von dem Hohlraum wegweisenden Seite zwischen dem Blendenelement und dem Maskenkörper gehaltert ist. Ein solcher Aufbau ermöglicht eine sehr einfache Montage der Elemente des Notatemventils und des Exspirationsventils an dem Maskenkörper, da das Blendenelement bei dessen Montage gleichzeitig den Verschlusskörper des Exspirationsventils fixiert. Wenn außerdem auch das Blendenelement als separates Bauteil ausgeführt ist, sind die Elemente, die die Funktion des Notatemventils bereitstellen, vollkommen von denen getrennt, die das Exspirationsventil bilden.

Hierbei ist es weiter bevorzugt, wenn das Exspirationsventil zwei getrennte, an das Blendenelement angrenzende Exspirationsventilöffnungen aufweist, wobei der Verschlusskörper einstückig ausgebildet ist und zwei sich von dem Blendenelement weg erstreckende Laschen aufweist. Dies ermöglicht zum einen eine einfache Montage der Elemente des Notatemventils und des Exspirationsventils vorzusehen, wobei zum anderen sichergestellt ist, dass der Öffnungsquerschnitt des Exspirationsventils einerseits hinreichend groß ist, aber andererseits von dem Verschlusselement zuverlässig verschlossen werden kann, weil die Flächen der Laschen des Verschlusselements vergleichsweise klein gewählt werden können, sich in Summe aber dennoch eine große Querschnittsfläche der Öffnung ergibt.

Dabei ist der Verschlusskörper des Exspirationsventils vorzugsweise derart ausgestaltet, dass er außerdem eine zentrale Öffnung aufweist, durch die sich das Blendenelement teilweise hindurch erstreckt und über der die Blendenöffnung angeordnet ist, sodass der Verschlusskörper dadurch zuverlässig an dem Maskenkörper festgeklemmt werden kann.

Hierbei ist es schließlich weiter bevorzugt, den Verschlusskörper symmetrisch auszugestalten, wobei dann die Exspirationsventilöffnungen und die Laschen des Verschlusskörpers auf gegenüberliegenden Seiten des Blendenelements angeordnet sind.

Schließlich kann in einer weiteren bevorzugten Ausführungsform der Maskenkörper der Atemmaske derart aufgebaut sein, dass er einen Hauptabschnitt aus einem flexiblen Material aufweist, innerhalb dessen ein Ventilabschnitt aus Hartplastik vorgesehen ist, der die Ventilanordnung aufnimmt. Der Hauptabschnitt ist dabei aus einem flexiblen Material gebildet, das sich gut an die Form des Mund- und Nasenabschnitts des Patienten anpasst, während der Ventilabschnitt aufgrund von dessen Steifigkeit die Montage erleichtert und ein zuverlässiges Abdichten besonders des Exspirationsventils ermöglicht.

Gemäß einem zweiten Aspekt der vorliegenden Erfindung wird ein Beatmungssystem mit einer zuvor beschriebenen Atemmaske vorgesehen, wobei der Zuführanschluss mit einer Atemgaszuführeinrichtung verbunden ist.

Insbesondere kann die Atemgaszuführeinrichtung einen Gasbeutel aufweisen, der mit einem Anschlussstück versehen ist, das mit dem Zuführanschluss verbunden ist, wobei das Anschlussstück ein Einatemventil um Steuern des Durchlasses von Gas von dem Anschlussstück in den Hohlraum aufweist und wobei das Einatemventil derart angepasst ist, dass es öffnet, wenn der Druck in dem Hohlraum um einen vorgegebenen Betrag unter dem Druck in dem Anschlussstück liegt. Ferner kann das Anschlussstück einen Anschluss für eine Atemgasversorgungsleitung aufweisen.

Im Folgenden wird die vorliegende Erfindung anhand einer lediglich ein bevorzugtes Ausführungsbeispiel zeigenden Zeichnung erläutert, wobei
- Fig. 1: das Ausführungsbeispiel einer erfindungsgemäßen Atemmaske in einer perspektivischen Ansicht zeigt,
- Fig. 2: eine Kombination des Ausführungsbeispiels aus Fig. 1 mit einer Atemgaszuführeinrichtung zeigt,
- Fig. 3: das Ausführungsbeispiel der Atemmaske aus Fig. 1 ohne Blendenelement, Verschlusselement und Verschlusskörper in perspektivischer Ansicht von vorne zeigt,
- Fig. 4: den Sauerstoffbeutel der Atemgaszuführeinrichtung getrennt von der Atemgasmaske zeigt,
- Fig. 5: das Blendenelement zusammen mit dem Verschlusselement und dem Verschlusskörper des Exspirationsventils des Ausführungsbeispiels aus den Fig. 1 und 3 in perspektivischer Ansicht zeigt,
- Fig. 6: eine Explosionsdarstellung des Blendenelements, des Verschlusselements und des Verschlusskörpers des Exspirationsventils und zeigt,
- Fig. 7: eine alternative Ausführungsform des Blendenelements, des Verschlusselements und des Verschlusskörpers für das Ausführungsbeispiel aus den Fig. 1 und 3 in einer ersten perspektivischer Ansicht zeigt,
- Fig. 8: die alternative Ausführungsform aus Fig. 7 in einer zweiten perspektivischen Ansicht zeigt,
- Fig. 9: die alternative Ausführungsform aus Fig. 7 in Draufsicht zeigt und
- Fig. 10: einen Schnitt entlang der Linie X-X aus Fig. 9 zeigt.

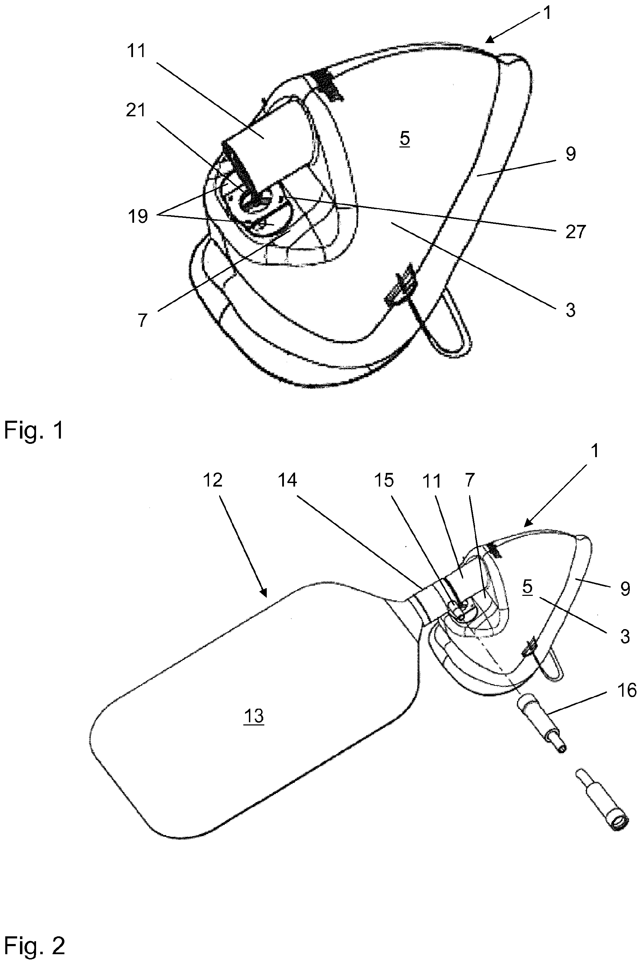

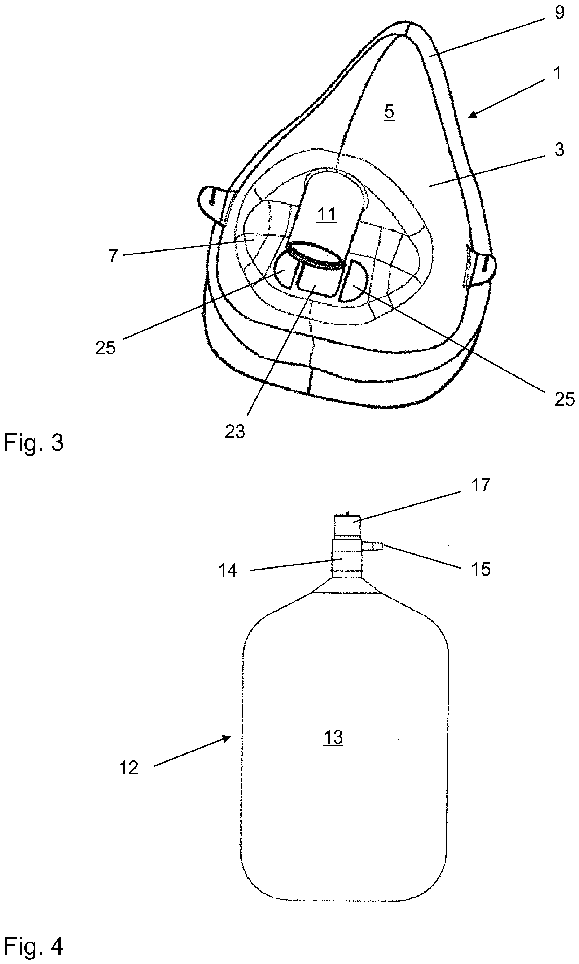

Wie insbesondere aus den Figuren 1 und 3 hervorgeht, weist das Ausführungsbeispiel einer erfindungsgemäßen Atemmaske 1 einen Maskenkörper 3 auf, der zweiteilig ausgebildet ist, wobei er einen Hauptabschnitt 5, der aus einem flexiblen Material gebildet ist, und einen darin angeordneten, aus Hartplastik gebildeten Ventilabschnitt 7 umfasst. Der Maskenkörper 3 und insbesondere der Hauptabschnitt 5 sind derart geformt, dass darin ein Hohlraum ausgebildet ist, der angepasst ist, um Mund und Nase eines Patienten aufnehmen zu können, wobei sich der flexible Rand 9 des Hauptabschnitts 5 des Maskenkörpers 3 an das Gesicht des Patienten anlegt, sodass der Hohlraum gegenüber der Umgebung abgedichtet ist.

An dem Ventilabschnitt 7 ist zunächst ein Zuführanschluss 11 vorgesehen, über den der Hohlraum der Atemmaske 1 mit einer Atemgaszuführeinrichtung 12 verbunden werden kann, die einen Gas bzw. Sauerstoffbeutel 13 aufweist, der wiederum über ein Anschlussstück 14 verfügt. An dem Anschlussstück 14 ist ein Anschluss 15 für eine Atemgas- bzw. Sauerstoffversorgungsleitung 16 vorgesehen, durch die ein konstanter Volumenstrom von mit Sauerstoff angereichertem Atemgas dem Sauerstoffbeutel 13 zugeführt wird.

In dem Anschlussstück 14 auf der von dem Sauerstoffbeutel 13 abgewandten Seite des Anschlusses 15 ist ferner ein Einatemventil 17 zum Steuern des Durchlasses von Gas von dem Anschlussstück 14 in den Hohlraum vorgesehen. Das Einatemventil 17 ist dabei derart angepasst, dass es öffnet, wenn der Druck in dem Hohlraum um einen vorgegebenen Betrag unter dem Druck in dem Anschlussstück 14 liegt. Das Einatemventil 17 ist somit normalerweise geschlossen und öffnet dann, wenn der Patient beim Einatmen den Druck im Hohlraum absenkt.

Benachbart zu dem Zuführanschluss 11 sind in dem Ventilabschnitt 7 ein Exspirationsventil 19 sowie ein Notatemventil 21 angeordnet, sodass diese Ventile 19, 21 eine Ventilanordnung bilden, über die eine Gasverbindung zu dem Hohlraum in dem Maskenkörper 3 hergestellt werden kann. Dabei geht der Aufbau des Exspirationsventils 19 sowie des Notatemventils 21 im Detail aus den Figuren 3, 5 und 6 hervor.

Wie Figur 3 zeigt, sind in dem Ventilabschnitt 7 des Maskenkörpers 3 eine Notatemventilöffnung 23 sowie zwei benachbart dazu an gegenüberliegenden Kanten angeordnete Exspirationsventilöffnungen 25 ausgebildet. Wie Figur 1 zu entnehmen ist, ist in die Notatemventilöffnung 23 ein Blendenelement 27 eingesetzt, wobei das Blendenelement 27 auf der von dem Hohlraum abgewandten Seite des Maskenkörpers 3 an diesem anliegt und wobei zwischen dem Blendenelement 27 und dem Maskenkörper 3 bzw. dem Ventilabschnitt 7 ein eine zentrale Öffnung 29 aufweisender und einstückig ausgebildeter Verschlusskörper 31 eingeklemmt ist. Dieser Verschlusskörper 31 ist Teil des Exspirationsventils 19, und daran vorgesehene Laschen 33, die sich von gegenüberliegenden Kanten des Blendenelements 27 weg erstrecken, verschließen die Exspirationsventilöffnungen 25, in dem sie an dem Ventilabschnitt 7 bzw. dem Maskenkörper 3 auf der von dem Hohlraum wegweisenden Seite anliegen.

Die zentrale Öffnung 29 in dem Verschlusskörper 31 ist außerdem derart angeordnet, dass sie über der Notatemventilöffnung 23 und einer zentralen Blendenöffnung 35 in dem Blendenelement 27 angeordnet ist. Die Blendenöffnung 35 bildet somit eine Gasverbindung zwischen dem Hohlraum um der die Atemmaske 1 umgebenden Atmosphäre.

In der Blendenöffnung 35 sind drei sich radial vom Zentrum der Blendenöffnung 35 weg erstreckende Arme 37 vorgesehen, die eine zentrale Aufnahmebuchse 39 halten, in der eine als Bohrung 41 ausgeführte Aufnahmeöffnung vorgesehen ist. Auf der zu dem Hohlraum weisenden Seite des Blendenelements 27 ist das Verschlusselement 43 des Notatemventils vorgesehen, das kreisflächenförmig ausgebildet ist und eine zu dem Blendenelement 27 weisende und im verschlossenen Zustand an diesem anliegende kreisringförmige Verschlussfläche 45 aufweist. Mittig am Verschlusselement 43 und von der Verschlussfläche 45 umgeben erstreckt sich ein als Stift 47 ausgebildetes erstes Verbindungselement senkrecht zu der Blendenöffnung 35 und davon sowie vom Verschlusselement 43 weg, das vorgesehen ist, mit dem als Bohrung 41 oder Aufnahmeöffnung in der Aufnahmebuchse 39 ausgebildeten zweiten Verbindungselement in dem Blendenelement 27 einzugreifen. Der Stift 47 weist einen ersten Abschnitt 49 auf, an den sich ein Konus 51 anschließt, dessen Außendurchmesser sich von dem ersten Abschnitt 49 weg verjüngt, wobei zwischen dem Konus 51 und dem ersten Abschnitt 49 ein Hinterschnitt ausgebildet ist. An den Konus 51 wiederum schließt sich ein zweiter Abschnitt 53 des Stifts 47 an.

Bei der Montage werden der zweite Abschnitt 53 sowie der Konus 51 des Stifts 47 durch die Bohrung 41 hindurchgezogen, bis der Hinterschnitt zwischen Konus 51 und erstem Abschnitt 49 auf der von dem Hohlraum abgewandten Seite des Blendenelements 27 an diesem zur Anlage kommt und so das Verschlusselement 43 an dem Blendenelement 27 verriegelt ist. In diesem Zustand kann der zweite Abschnitt 53 des Stifts 47 von dem Konus abgetrennt werden, da der zweite Abschnitt 53 hier lediglich als Montagehilfe dient.

Die Anordnung aus Verschlusskörper 31, Blendenelement 27 und Verschlusselement 43 wird dann mit Hilfe der an dem Blendenelement 27 vorgesehenen Rastarme 55, die mit dem Rand der Notatemventilöffnung 23 in dem Ventilabschnitt 7 eingreifen, an dem Maskenkörper 3 befestigt, wobei dann das Blendenelement 27 den Verschlusskörper 31 an dem Maskenkörper 3 bzw. dem Ventilabschnitt 7 festklemmt.

Die Verschlussfläche 45 des als separates Bauteil ausgebildeten Verschlusselements 43 liegt normalerweise an dem Blendenelement 27 an. Wenn jedoch der Druck im Inneren des Hohlkörpers gegenüber der umgebenden Atmosphäre um einen hinreichend großen Betrag abgesenkt wird, der außerdem größer ist als der Betrag, der notwendig ist, das Einatemventil 17 zu öffnen, wird das Verschlusselement 43 von dem Blendenelement 27 weg gebogen, sodass die Verschlussfläche 45 von dem Blendenelement 27 abgehoben und ein Durchgang durch die Blendenöffnung 35 hindurch in den Hohlraum geschaffen wird und der Patient auf diesem Weg Umgebungsluft als Atemluft erhält.

Das Verschlusselement 43 ist damit derart flexibel ausgebildet, dass es zwischen einer Öffnungsstellung, in der die Verschlussfläche 45 von dem Blendenelement 27 beabstandet ist, sodass ein Strömen von Gas durch die Blendenöffnung 35 zugelassen wird, und einer Schließstellung verformt werden kann, in der in diesem Ausführungsbeispiel die Verschlussfläche 45 auf der zum Hohlraum weisenden Seite an dem Blendenelement 27 anliegt und die Blendenöffnung 35 zur die Atemmaske 1 umgebenden Atmosphäre abdeckt, sodass ein Strömen von Gas durch die Blendenöffnung 35 verhindert wird. Die Verschlussfläche 45 könnte in der Schließstellung bei anderen Abmessungen allerdings auch zur Anlage an den Maskenkörper 3 kommen. In einem solchen Fall würde das Blendenelement im Sinne der vorliegenden Erfindung dann auch von dem Maskenkörper gebildet.

Das aus dem Verschlusselement 43 und dem Blendenelement 27 gebildete Notatemventil 21 ist daher derart angepasst, dass der Druck im Hohlraum, der erforderlich ist, um einen Durchlass von Gas durch das Notatemventil 21 zuzulassen, niedriger ist, als der Druck im Hohlraum, der erforderlich ist, um einen Durchlass von Gas durch das Zuführanschluss 11 zuzulassen.

Dadurch, dass das Verschlusselement 43 als separates Bauteil ausgebildet ist, kann es, was das Material und die Abmessungen angeht, sehr genau angepasst werden, damit die zuvor erwähnte Bedingung, dass ein Öffnen nur dann erfolgt, wenn ein Druck vorliegt, der niedriger ist als der Druck, der benötigt wird, um das Einatemventil 17 zu öffnen, immer erfüllt ist. Wäre dies nicht der Fall, bestünde die Gefahr, dass der Patient auch dann über das Notatemventil 21 Atemgas bezieht, obwohl die Atemgasversorgung durch das Einatemventil 17 noch gegeben ist. Alternativ könnte außerdem das Problem auftreten, dass der Druck, der zum Öffnen des Notatemventils erforderlich ist, zu niedrig ist und der Patient einen zu großen Unterdruck erzeugen muss und es damit eine zu große Anstrengung darstellt, um mit Atemgas versorgt zu werden, wenn die Atemgasversorgung ausgefallen ist.

Außerdem wird durch die Anordnung des Blendenelements 27 und der Notatemventilöffnung 23 zwischen den beiden Exspirationsventilöffnungen 25 ein einfacher Aufbau des Exspirationsventils 19 ermöglicht, bei dem der einstückig ausgebildete Verschlusskörper 31 mit den beiden Laschen 33 durch das Blendenelement 27 geklemmt und damit gehaltert wird. Die Laschen 33 dichten normalerweise die Exspirationsventilöffnungen 25 ab. Wenn der Patient jedoch ausatmet und sich in dem Hohlraum ein Druck ausbildet, der um einen vorgegebenen Betrag über dem Druck der die Atemmaske 1 umgebenden Atmosphäre liegt, werden die Laschen 33 von den Exspirationsventilöffnungen 25 weg gebogen und diese freigegeben, sodass die ausgeatmete Luft aus dem Hohlraum des Maskenkörpers 3 entweichen kann.

Beim Einatmen öffnet normalerweise aufgrund des von dem Patienten im Hohlraum des Maskenkörpers 3 erzeugten Unterdrucks das Einatemventil 17, sodass beispielsweise mit Sauerstoff angereichertes Atemgas entweder aus dem Sauerstoffbeutel 13 oder direkt aus der Sauerstoffversorgungsleitung 16 zu dem Patienten gelangt. Atmet der Patient anschließend aus, bildet sich in dem Hohlraum ein Überdruck gegenüber der Umgebung, sodass in der Folge die Laschen 33 von dem Maskenkörper 3 bzw. dem Ventilabschnitt 7 abgehoben werden.

In dem Fall, dass das in der Sauerstoffversorgungsleitung 16 und dem Sauerstoffbeutel 13 bereitgestellte Atemgas nicht ausreicht, bildet sich im Hohlraum des Maskenkörpers 3 ein Druck aus, der niedriger ist als der, der benötigt wird, um das Einatemventil 17 zu öffnen. In diesem Fall öffnet dann auch das Notatemventil 21 in der Weise, dass sich die Verschlussfläche 45 von dem Blendenelement 27 abhebt. Somit ist sichergestellt, dass der Patient in jedem Fall Luft zum Atmen erhält.

Durch die erfindungsgemäße Ausgestaltung der Ventilanordnung und insbesondere der Kombination aus dem Notatemventil 21 und dem Exspirationsventil 19 wird zudem sichergestellt, dass die zuvor genannte Bedingung an das Öffnungsverhalten von Notatemventil 21 und Einatemventil 17 gegeben ist, aber dennoch eine einfache Montage ermöglicht wird.

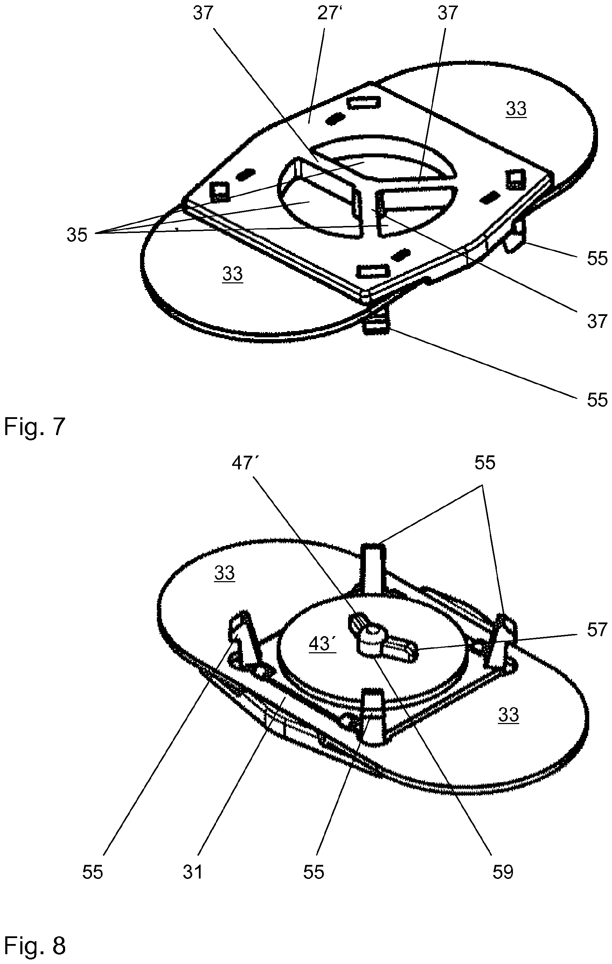

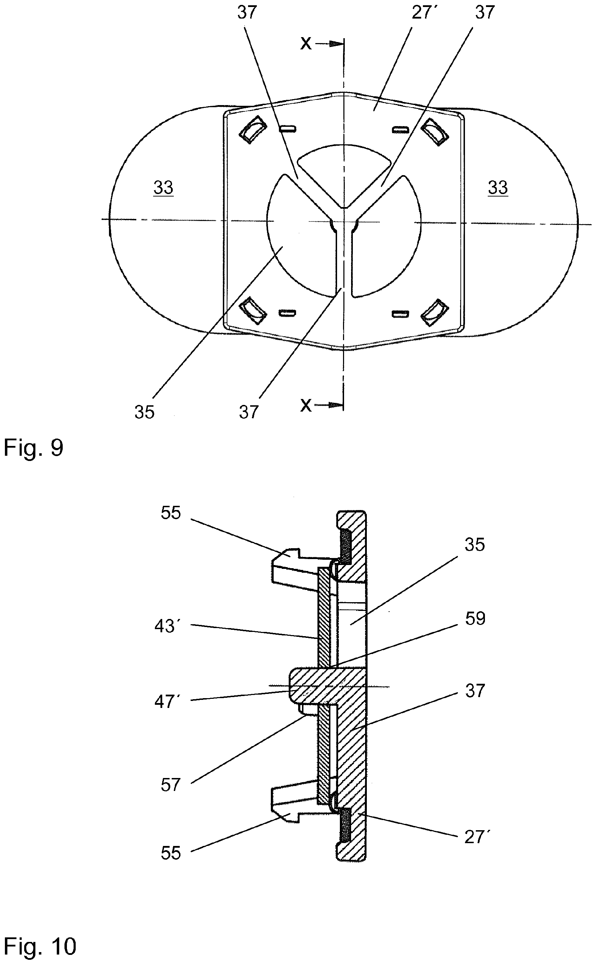

In den Figuren 7 bis 10 ist eine alternative Ausführungsform für die Kombination aus Blendenelement 27', Verschlusselement 43' und Verschlusskörper 31 mit Laschen 33 gezeigt, die ebenfalls in dem Ausführungsbeispiel der Atemmaske 1 gemäß den Fig. 1 bis 3 zur Anwendung kommen kann.

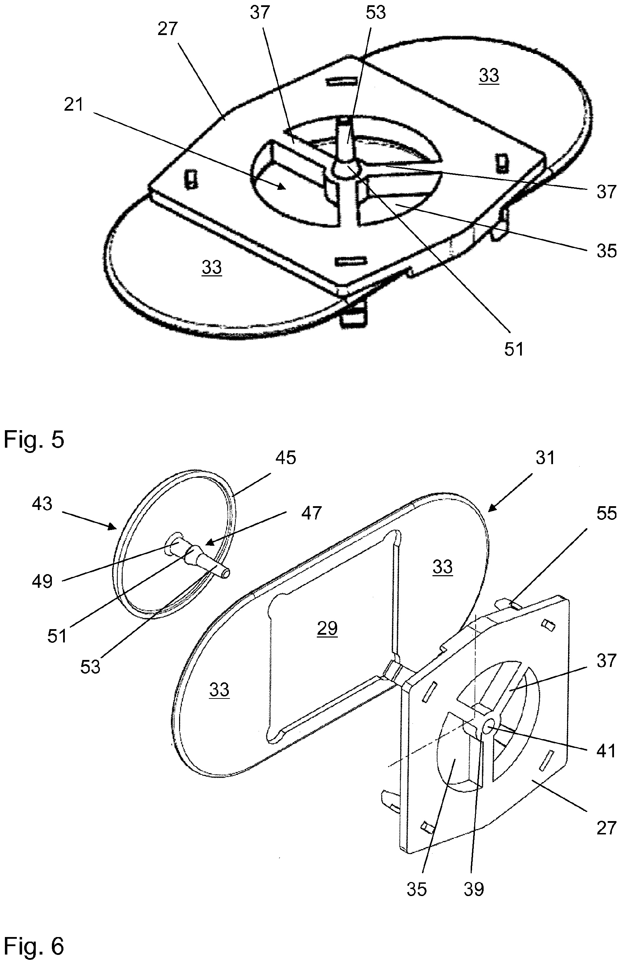

Diese Ausführungsform ist ähnlich der aus den Fig. 5 und 6 aufgebaut, sodass hier für identische Elemente identische Bezugszeichen verwendet werden, und weist ebenfalls ein mit einer Blendenöffnung 35 und Rastarmen 55 versehenes Blendenelement 27' auf, mit dem ein Verschlusskörper 31 mit Laschen 33 an dem Maskenkörper 3 festgeklemmt werden kann. In die Blendenöffnung 35 erstrecken sich von deren Rand Arme 37. An den Armen 37 ist das auch hier ein als separates Bauteil ausgebildetes Verschlusselement 43' befestigt, das ähnlich dem Verschlusselement 43 aus Fig. 6 ausgebildet ist.

Unterscheiden tut sich diese Ausführungsform von der aus den Fig. 5 und 6 dadurch, dass das Blendenelement 27' an den sich in die Blendenöffnung 35 erstreckenden Armen 37 einen sich senkrecht zur Blendenöffnung 35 davon weg erstreckenden Stift 47' aufweist, der sich zu dem Verschlusselement 43' hin erstreckt. Ferner ist das freie Ende des Stifts 47' ist mit einem Vorsprung 57 versehen.

Das Verschlusselement 43' wiederum ist im Unterschied zu der Ausführungsform aus den Fig. 5 und 6 mit einer Aufnahmeöffnung 59 versehen, durch die sich der Stift 47' mit dem Vorsprung 57 hindurch erstreckt und dadurch das Verschlusselement 43' am Blendenelement 27' festlegt.

Bei dieser Ausführungsform ist also das erste Verbindungselement an dem Verschlusselement 43' als Aufnahmeöffnung 59 ausgebildet, während das am Blendenelement 27' vorgesehene zweite Verbindungselement als Stift 47' ausgestaltet ist.

Aber auch bei dieser Ausführungsform werden die schon im Zusammenhang mit der ersten Ausführungsform erläuterten Vorteile erreicht, da auch hier der Verschlusskörper 43' als separates Bauteil der Ventilanordnung ausgebildet ist.

## Patentansprüche

1. Atemmaske (1) mit einem Maskenkörper (3), der einen zu einer Seite offenen Hohlraum umgibt und der zur Anlage an ein menschliches Gesicht um dessen Mund und/oder Nase vorgesehen ist, wobei Mund und/oder Nase in dem Hohlraum angeordnet werden können,
mit einem Zuführanschluss (11) zum Anschluss einer Atemgaszuführeinrichtung (12) für eine Zufuhr von Gas in den Hohlraum,
mit einer Ventilanordnung, die ein Exspirationsventil (19) zum Steuern des Durchlasses von Gas aus dem Hohlraum in die die Atemmaske (1) umgebende Atmosphäre und ein Notatemventil (21) zum Steuern des Durchlasses von Gas aus der die Atemmaske (1) umgebenden Atmosphäre in den Hohlraum aufweist,
wobei das Exspirationsventil (19) einen Verschlusskörper (31) zum Verschließen einer Exspirationsventilöffnung (25) aufweist und das Exspirationsventil (19) derart angepasst ist, dass es öffnet, wenn der Druck im Hohlraum um einen vorgegebenen Betrag über dem Druck der die Atemmaske (1) umgebenden Atmosphäre liegt,
wobei das Notatemventil (21) derart angepasst ist, dass es öffnet, wenn der Druck im Hohlraum um einen vorgegebenen Betrag unter dem Druck der die Atemmaske (1) umgebenden Atmosphäre liegt,
wobei das Notatemventil (21) ein Blendenelement (27, 27') aufweist, das mit einer Blendenöffnung (35) versehen ist, die eine Gasverbindung zwischen dem Hohlraum und der die Atemmaske (1) umgebenden Atmosphäre bildet,
wobei das Notatemventil (21) ein Verschlusselement (43, 43') mit einer Verschlussfläche (45) aufweist, das derart flexibel ausgebildet ist, dass es zwischen einer Öffnungsstellung, in der die Verschlussfläche (45) von dem Blendenelement (27, 27') beabstandet ist, sodass ein Strömen von Gas durch die Öffnung (35) zugelassen wird, und einer Schließstellung verformbar ist, in der die Verschlussfläche (45) an dem Blendenelement (27, 27') anliegt, sodass das Verschlusselement (43, 43') die Blendenöffnung (35) zur die Atemmaske (1) umgebenden Atmosphäre verschließt und ein Strömen von Gas durch die Blendenöffnung (35) verhindert wird,
**dadurch gekennzeichnet,**
**dass** das Verschlusselement (43, 43') als separates Bauteil der Ventilanordnung ausgebildet ist,
**dass** das Blendenelement (27, 27') als separates Bauteil ausgebildet ist, das auf der von dem Hohlraum Weg weisenden Seite an dem Maskenkörper (3) befestigt ist und
**dass** der Verschlusskörper (31) des Exspirationsventils (19) auf der von dem Hohlraum Weg weisenden Seite zwischen dem Blendenelement (27, 27') und dem Maskenkörper (3) gehaltert ist.

2. Atemmaske nach Anspruch 1, wobei das Verschlusselement (43, 43') ein erstes Verbindungselement (47, 59) aufweist und
wobei das Blendenelement (27, 27') ein zweites Verbindungselement (41, 47') zum Eingriff mit dem ersten Verbindungselement (47, 59) aufweist.

3. Atemmaske (1) nach Anspruch 2, wobei das erste Verbindungselement von der Verschlussfläche (45) umgeben ist,
wobei das zweite Verbindungselement innerhalb der Blendenöffnung (35) angeordnet ist und einen sich radial zum Rand der Blendenöffnung (35) erstreckenden Arm (37) aufweist,
wobei eines der Verbindungselemente als sich von der Blendenöffnung (35) weg erstreckender Stift (47, 47') ausgebildet ist und
wobei das andere der Verbindungselemente als Aufnahmeöffnung (41, 59) für einen Eingriff mit dem Stift (47, 47') ausgebildet ist.

4. Atemmaske (1) nach einem der Ansprüche 1 bis 3, wobei das Exspirationsventil (19) zwei getrennte, an das Blendenelement (27, 27') angrenzende Exspirationsventilöffnungen (25) aufweist und
wobei der Verschlusskörper (31) einstückig ausgebildet ist und zwei sich von dem Blendenelement (27, 27') weg erstreckende Laschen (33) aufweist.

5. Atemmaske (1) nach einem der Ansprüche 1 bis 4, wobei der Verschlusskörper (31) eine zentrale Öffnung (29) aufweist, über der die Blendenöffnung (35) angeordnet ist.

6. Atemmaske (1) nach einem der Ansprüche 4 oder 5, wobei die Exspirationsventilöffnungen (25) und die Laschen (33) des Verschlusskörpers (31) auf gegenüberliegenden Seiten des Blendenelements (27, 27') angeordnet sind.

7. Atemmaske (1) nach einem der Ansprüche 1 bis 6, wobei der Maskenkörper (3) einen Hauptabschnitt (5) aus einem flexiblen Material und einen Ventilabschnitt (7), in dem die Ventileinheit vorgesehen ist und der aus einem steifen Material, vorzugsweise einem Hartplastikmaterial, gebildet ist, aufweist und
wobei der Hauptabschnitt (5) den Ventilabschnitt (7) umgibt.

8. Beatmungssystem mit einer Atemmaske nach einem der Ansprüche 1 bis 7 und mit einer mit dem Zuführanschluss (11) verbundenen Atemgaszuführeinrichtung (12).

9. Beatmungssystem nach Anspruch 8, wobei die Atemgaszuführeinrichtung einen Gasbeutel (13) aufweist, der mit einem Anschlussstück (14) versehen ist, das mit dem Zuführanschluss (11) verbunden ist,
wobei das Anschlussstück (14) ein Einatemventil (17) zum Steuern des Durchlasses von Gas von dem Anschlussstück (14) in den Hohlraum aufweist und
wobei das Einatemventil (17) derart angepasst ist, dass es öffnet, wenn der Druck in dem Hohlraum um einen vorgegebenen Betrag unter dem Druck in dem Anschlussstück (14) liegt.

10. Beatmungssystem nach Anspruch 9, wobei das Anschlussstück einen Anschluss (15) für eine Atemgasversorgungsleitung (16) aufweist.

## Claims

1. A breathing mask (1) comprising a mask body (3) which surrounds a cavity that is open towards one side and which is intended for coming into contact with a human face around the mouth and/or nose thereof, wherein the mouth and/or nose may be arranged in the cavity,
a feed port (11) for connecting a breathing gas feed device (12) for feeding gas into the cavity;
a valve arrangement comprising an exhalation valve (19) for controlling the flow of gas from the cavity into atmosphere surrounding the breathing mask (1) and an emergency breathing valve (21) for controlling the flow of gas from the atmosphere surrounding the breathing mask into the cavity,
wherein the exhalation valve (19) comprises a closing body (31) for closing an exhalation valve opening (25) and wherein the exhalation valve (19) is adapted such that the exhalation valve opens when the pressure in the cavity is above the pressure of the atmosphere surrounding the breathing mask (1) by a preset value,
wherein the emergency breathing valve (21) is adapted such that it opens when pressure in the cavity is below pressure of the atmosphere surrounding the breathing mask (1) by a preset value,
wherein the emergency breathing valve (21) comprises a diaphragm element (27, 27') which is provided with a diaphragm opening (35) which forms a gas connection between the cavity and the atmosphere surrounding the breathing mask (1),
wherein the emergency breathing valve (21) comprises a closing element (43, 43') with a closing surface (45), which closing element is flexible such that it is deformable between an open position in which the closing surface (45) is spaced apart from the diaphragm element (27, 27'), so that flow of gas through the opening (35) is allowed, and a closed position in which the closing surface (45) is in contact with the diaphragm element (27, 27') so that the closing element (43, 43') closes the diaphragm opening (35) to the atmosphere surrounding the breathing mask (1) and flow of gas through the diaphragm opening (35) is prevented,
**characterized**
**in that** the closing element (43, 43') is formed as a separate component of the valve arrangement,
in the that the diaphragm element (27, 27') is formed as a separate component which is fastened to the mask body (3) on the side pointing away from the cavity, and
**in that** the closing body (31) of the exhalation valve (19) is held on the side pointing away from the cavity between the diaphragm element (27, 27') and the mask body (3).

2. A breathing mask according to claim 1, wherein the closing element (43, 43') comprises a first connection element (47, 59) and
wherein the diaphragm element (27, 27') comprises a second connection element (41, 47') for meshing with the first connection element (47, 59).

3. A breathing mask (1) according to claim 2, wherein the first connection element is surrounded by the closing surface (45),
wherein the second connection element is arranged within the diaphragm opening (35) and comprises an arm (37) extending radially to the edge of the diaphragm opening (35),
wherein one of the connection elements comprises a pin (47, 47') extending away from the diaphragm opening (35) and
wherein the other of the connection elements is formed as a receiving opening (41, 59) for meshing with the pin (47, 47').

4. A breathing mask (1) according to any of the claims 1 to 3, wherein the exhalation valve (19) comprises two separate exhalation valve openings (25) adjoining the diaphragm element (27, 27') and
wherein the closing body (31) is formed as one piece and has two flaps (33) extending away from the diaphragm element (27, 27').

5. A breathing mask (1) according to any of the claims 1 to 4, wherein the closing body (31) has a central opening (29) above which the diaphragm opening (35) is arranged.

6. A breathing mask (1) according to any of the claims 4 or 5, wherein the exhalation valve openings (25) and the flaps (33) of the closing body (31) are arranged on opposite sides of the diaphragm element (27, 27').

7. A breathing mask (1) according to any of the claims 1 to 6, wherein the mask body (3) has a main section (5) made of a flexible material and a valve section (7) in which the valve assembly is provided and which is formed form a rigid material, preferably from a hard plastic material, and
wherein the main section (5) surrounds the valve section (7) .

8. A ventilation system comprising a breathing mask according to any of the claims 1 to 7, and a breathing gas feed device (12) connected to the feed port (11).

9. A breathing system according to claim 8, wherein the breathing gas feed device has a gas bag (13) which is provided with a connection member (14) which is connected to the feed port (11),
wherein the connection member (14) has an inhalation valve (17) for controlling the flow of gas from the connection member (14) into the cavity and
wherein the inhalation valve (17) is adapted such that it opens when the pressure in the cavity is below the pressure present in the connection member (14) by a preset valve.

10. A breathing system according to claim 9, wherein the connection member comprises a connector (15) for a breathing gas supply line (16).

## Revendications

1. Masque respiratoire (1) avec un corps de masque (3) qui entoure une cavité ouverte d'un côté et qui est prévu pour l'appui contre un visage humain autour de sa bouche et/ou de son nez, dans lequel la bouche et/ou le nez peuvent être agencés dans la cavité,
avec un raccord d'amenée (11) pour le raccordement d'un dispositif d'amenée de gaz respiratoire (12) pour une amenée de gaz dans la cavité,
avec un agencement de soupape qui présente une soupape d'expiration (19) pour la commande du passage de gaz hors de la cavité dans l'atmosphère entourant le masque respiratoire (1) et une soupape de respiration de secours (21) pour la commande du passage de gaz hors de l'atmosphère entourant le masque respiratoire (1) dans la cavité,
dans lequel la soupape d'expiration (19) présente un corps de fermeture (31) pour la fermeture d'une ouverture de soupape d'expiration (25) et la soupape d'expiration (19) est adaptée de telle manière qu'elle s'ouvre lorsque la pression dans la cavité se trouve d'une valeur prescrite au-dessus de la pression de l'atmosphère entourant le masque respiratoire (1),
dans lequel la soupape de respiration de secours (21) est adaptée de telle manière qu'elle s'ouvre lorsque la pression dans la cavité se trouve d'une valeur prescrite sous la pression de l'atmosphère entourant le masque respiratoire (1),
dans lequel la soupape de respiration de secours (21) présente un élément de diaphragme (27, 27') qui est pourvu d'une ouverture de diaphragme (35) qui forme une liaison de gaz entre la cavité et l'atmosphère entourant le masque respiratoire (1),
dans lequel la soupape de respiration de secours (21) présente un élément de fermeture (43, 43') avec une surface de fermeture (45) qui est réalisée de manière flexible de telle manière qu'elle soit déformable entre une position d'ouverture dans laquelle la surface de fermeture (45) est espacée de l'élément de diaphragme (27, 27') de sorte qu'un flux de gaz au travers de l'ouverture (35) soit autorisé, et une position de fermeture dans laquelle la surface de fermeture (45) repose contre l'élément de diaphragme (27, 27') de sorte que l'élément de fermeture (43, 43') ferme l'ouverture de diaphragme (35) par rapport à l'atmosphère entourant le masque respiratoire (1) et un flux de gaz au travers de l'ouverture de diaphragme (35) soit empêché,
**caractérisé en ce**
**que** l'élément de fermeture (43, 43') est réalisé en tant que composant séparé de l'agencement de soupape,
**que** l'élément de diaphragme (27, 27') est réalisé en tant que composant séparé qui est fixé sur le côté éloigné de la cavité sur le corps de masque (3) et
**que** le corps de fermeture (31) de la soupape d'expiration (19) est maintenu sur le côté éloigné de la cavité entre l'élément de diaphragme (27, 27') et le corps de masque (3).

2. Masque respiratoire selon la revendication 1, dans lequel l'élément de fermeture (43, 43') présente un premier élément de liaison (47, 59) et
dans lequel l'élément de diaphragme (27, 27') présente un second élément de liaison (41, 47') pour la mise en prise avec le premier élément de liaison (47, 59).

3. Masque respiratoire (1) selon la revendication 2, dans lequel le première élément de liaison est entouré de la surface de fermeture (45),
dans lequel le second élément de liaison est agencé dans l'ouverture de diaphragme (35) et présente un bras (37) s'étendant radialement par rapport au bord de l'ouverture de diaphragme (35),
dans lequel un des éléments de liaison est réalisé en tant que tige (47, 47') s'étendant loin de l'ouverture de diaphragme (35) et
dans lequel l'autre des éléments de liaison est réalisé en tant qu'ouverture de réception (41, 59) pour une mise en prise avec la tige (47, 47').

4. Masque respiratoire (1) selon l'une des revendications 1 à 3, dans lequel la soupape d'expiration (19) présente deux ouvertures de soupape d'expiration (25) séparées contigües à l'élément de diaphragme (27, 27') et
dans lequel le corps de fermeture (31) est réalisé d'un seul tenant et présente deux languettes (33) s'étendant loin de l'élément de diaphragme (27, 27').

5. Masque respiratoire (1) selon l'une des revendications 1 à 4, dans lequel le corps de fermeture (31) présente une ouverture centrale (29) au-dessus de laquelle l'ouverture de diaphragme (35) est agencée.

6. Masque respiratoire (1) selon l'une quelconque des revendications 4 ou 5, dans lequel les ouvertures de soupape d'expiration (25) et les languettes (33) du corps de fermeture (31) sont agencées sur des côtés opposés de l'élément de diaphragme (27, 27').

7. Masque respiratoire (1) selon l'une quelconque des revendications 1 à 6, dans lequel le corps de masque (3) présente une section principale (5) en un matériau flexible et une section de soupape (7) dans laquelle l'unité de soupape est prévue et qui est formée en un matériau rigide, de préférence un matériau en plastique dur et
dans lequel la section principale (5) entoure la section de soupape (7).

8. Système de respiration avec un masque respiratoire selon l'une quelconque des revendications 1 à 7 et avec un dispositif d'amenée de gaz respiratoire (12) raccordé au raccord d'amenée (11).

9. Système de respiration selon la revendication 8, dans lequel le dispositif d'amenée de gaz respiratoire présente une poche de gaz (13) qui est pourvue d'une pièce de raccord (14) qui est raccordée au raccord d'amenée (11),
dans lequel la pièce de raccord (14) présente une soupape de respiration inspiratoire (17) pour la commande du passage de gaz de la pièce de raccord (14) dans la cavité et
dans lequel la soupape de respiration inspiratoire (17) est adaptée de telle manière qu'elle s'ouvre lorsque la pression dans la cavité se trouve d'une valeur prescrite sous la pression dans la pièce de raccord (14).

10. Système de respiration selon la revendication 9, dans lequel la pièce de raccord présente un raccord (15) pour une conduite d'alimentation en gaz respiratoire (16).
